(19) **Europäisches Patentamt**
**European Patent Office**
**Office européen des brevets**

(11) **EP 4 741 006 A1**

(12) # EUROPEAN PATENT APPLICATION

(43) Date of publication:
**13.05.2026 Bulletin 2026/20**

(21) Application number: **25213874.8**

(22) Date of filing: **06.11.2025**

(51) International Patent Classification (IPC):
**A61N 5/10** (2006.01) **G06N 20/00** (2019.01)
**G16H 20/40** (2018.01)

(52) Cooperative Patent Classification (CPC):
**A61N 5/1031; G06N 3/006; G06N 3/092; G06N 7/01; G06N 20/00;** A61N 2005/1041; G16H 20/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **11.11.2024 US 202418943674**

(71) Applicant: **Siemens Healthineers International AG 6312 Steinhausen (CH)**

(72) Inventors:
• **BASIRI, Shahab**
 **02570 Siuntio (FI)**
• **CZEIZLER, Elena**
 **00390 Helsinki (FI)**

(74) Representative: **Mathisen & Macara LLP Charta House 30-38 Church Street Staines-upon-Thames TW18 4EP (GB)**

(54) **SYSTEMS AND METHODS FOR AUTOMATIC RADIOTHERAPY TREATMENT PLAN GENERATION USING REINFORCEMENT LEARNING BOOSTED WITH A MACHINE LEARNING DOSE PREDICTION MODEL**

(57) Embodiments described herein provide for radiotherapy treatment plan generation using reinforcement learning. A processor can use (202) a machine learning model (e.g., a neural network, random forest, a support vector machine, etc.) to predict a three-dimensional dose distribution based on the patient's treatment attributes. The processor can generate (204) a cost function with weighted dose-volume objectives from the predicted three-dimensional dose distribution. The processor can determine (206) a first three-dimensional dose distribution that reduces a first cost value based on the weighted dose-volume objectives. The processor can determine (208) a difference between the predicted and determined dose distributions. The processor can adjust (210), using a reinforcement learning agent, the dose-volume objectives of the cost function. The processor can reduce (212) a cost value of the adjusted cost function to a second cost value. If (214) the difference between the first and second cost values meets a given threshold, the processor can generate (216) a radiotherapy treatment plan.

**FIG. 2**

## Description

### TECHNICAL FIELD

This application relates generally to generating a radiotherapy treatment plan using reinforcement learning.

### BACKGROUND

Radiation therapy treatment planning (RTTP) is a complex process that contains specific guidelines, protocols, and instructions adopted by different medical professionals, such as the clinicians, the medical device manufacturers, and the like. Typically, identifying and applying guidelines to implement radiation therapy treatment are performed by complex computer models that receive treatment objectives from a treating physician and identify suitable attributes of the RTTP. For instance, the treating physicians may identify the treatment modality (e.g., choose between the volumetric modulated arc therapy (VMAT) or intensity-modulated radiation therapy (IMRT)). The treating physician may then input various objectives and goals to be achieved via the treatment, such as dose objectives to be achieved for one or more structures of the patient. A software solution may then use various methods to calculate attributes of the patient's treatment, such as determining beam-limiting device angles and radiation-emitting attributes. In the case of IMRT, the beam delivery directions and number of beams are the specifically relevant variables that must be decided, whereas, for VMAT, the software solution may need to choose the number of arcs and their corresponding start and stop angles.

In personalized radiation therapy plan optimization, achieving and/or scoring the trade-offs between target coverage and OAR sparing heavily depends on the formulation of the cost function. Formulating the cost function can require case-specific optimization objectives, which are not known to the planner prior to the optimization. Therefore, the planner needs to find the objectives through an iterative process. Accordingly, generating a personalized plan can be a time-consuming and resource-intensive process, even if all of the components in the plan generation pipeline are fully automated.

### SUMMARY

In accordance with a first aspect of the invention, there is provided a method as defined by claim 1.

In accordance with a second aspect of the invention, there is provided a system as defined by claim 9.

Optional features are defined by the dependent claims.

A computer model can be configured to generate radiotherapy treatment plans using a cost function to determine radiation dose distributions among patient structures. A user can input initial objectives for the cost function, and the computer model can iteratively adjust the objectives to identify or determine an optimal dose distribution for treating a patient. This process can involve a large amount of time and computer resources depending on how close the initial objectives are to the optimal dose distribution and/or the number of iterations of adjustments the computer model performs to identify the optimal dose distribution.

A computer implementing the systems and methods described herein can use machine learning and reinforcement learning techniques to improve efficiency in generating a radiotherapy treatment plan. The computer can do so using a reinforcement learning model and a dose prediction machine learning model. For example, the computer can receive patient treatment attributes (e.g., computed tomography (CT) images, field geometry settings, dose prescriptions, etc.) and use the treatment attributes as input into the dose prediction machine learning model. The computer can execute the dose prediction machine learning model to generate a predicted three-dimensional dose distribution. The computer can use the predicted three-dimensional dose distribution to create a cost function with weighted objectives for different patient structures (e.g., organs, bones, tumors, etc.). The computer can execute or apply an optimization algorithm on the cost function containing the objectives to generate a first three-dimensional dose distribution that reduces (e.g., minimizes) the cost function to a first cost value of implementing the first three-dimensional dose distribution (e.g., treating the patient using the first three-dimensional dose distribution).

The computer can implement a reinforcement learning agent to adjust the objectives' values and/or weights of the cost function to identify optimal objectives which can be used to generate an optimal plan for treating the patient. For example, the reinforcement learning agent can determine a difference (e.g., determine a distance using a distance function) between the first three-dimensional dose distribution and the predicted three-dimensional dose distribution. The reinforcement learning agent can adjust the objectives' values and/or weights of the cost function based on the difference, such as to make the three-dimensional dose distribution of the cost function closer to the predicted three-dimensional dose distribution. The reinforcement learning agent can further adjust the objectives' values and/or weights of the cost function according to one or more rules (e.g., defined rules) that may correspond to generating objectives that improve on objectives that result in the predicted three-dimensional dose distribution. The computer can apply or execute the adjusted cost function to generate a second three-dimensional dose distribution that reduces the cost function to a second cost value of implementing the second three-dimensional dose distribution. The computer can compare the sequentially determined cost values to determine whether a difference between several consecutive cost values satisfies (e.g., is less than) a threshold, or otherwise converges. Responsive to determining the difference does not satisfy the threshold, the computer can repeat the process using the reinforcement learning agent until determining sequentially generated cost values for the cost function satisfy the threshold or converge. The computer can use the final objectives in the patient's radiotherapy treatment plan. Using a reinforcement learning model in combination with a dose prediction machine learning model in this way can reduce latency and processing resources by starting closer to optimal objectives (e.g., optimal objectives for the individuals for which the reinforcement learning agent is generating the radiotherapy treatment plan) and requiring fewer iterations of adjustments to the cost function than other methods.

In some cases, the reinforcement learning agent can additionally determine whether other criteria are met before determining the objectives are finalized or the objectives have converged. For example, the reinforcement learning agent can determine the mean dose levels delivered to defined organs at risk are below a threshold and/or determine whether target coverage metrics (which may not be explicitly included in the cost function) satisfy defined objectives. If, after several iterations, the reinforcement learning agent determines that there is no improvement in these metrics, the reinforcement learning agent can determine that the cost function has converged (e.g., instead of or in addition to determining the objectives of the cost function has converged).

In an embodiment, a method includes executing, by a processor, a dose prediction machine learning model using one or more treatment attributes for a patient to generate a predicted three-dimensional dose distribution for the patient; generating, by the processor, a set of weighted dose-volume objectives of a cost function based on the predicted three-dimensional dose distribution for the patient; determining, by the processor, a first three-dimensional dose distribution that reduces a first cost value of the cost function; determining, by the processor, a difference between the first three-dimensional dose distribution and the predicted three-dimensional dose distribution; adjusting, by the processor using a reinforcement learning agent, the set of weighted dose-volume objectives of the cost function based on the difference between the first three-dimensional dose distribution and the predicted three-dimensional dose distribution; determining, by the processor, a second three-dimensional dose distribution that reduces a second cost value based on the adjusted set of weighted dose-volume objectives of the cost function; and responsive to determining a difference between the first cost value and the second cost values satisfies a threshold, generating, by the processor, a radiotherapy treatment plan for the patient based on the adjusted set of weighted dose-volume objectives.

The processor can train the reinforcement learning agent based on training data from a plurality of different patients. For instance, for an individual patient, the method may further include executing, by the processor, the dose prediction machine learning model using one or more second treatment attributes for a second patient to generate a second predicted three-dimensional dose distribution for the second patient; generating, by the processor, a second set of weighted dose-volume objectives of a second cost function based on the second predicted three-dimensional dose distribution for the second patient; determining, by the processor, a third three-dimensional dose distribution that reduces a third cost value of the second cost function; determining, by the processor, a second difference between the third three-dimensional dose distribution and the second predicted three-dimensional dose distribution; determining, by the processor, a reward value at least according to the difference between the third three-dimensional dose distribution and the second predicted three-dimensional dose distribution; and training, by the processor, the reinforcement learning agent based on the reward value.

The method may further include receiving, by the processor, third one or more treatment attributes of a third radiotherapy treatment plan for a third patient; generating, by the processor, a third set of weighted dose-volume objectives based on the third one or more treatment attributes for the third patient; executing, by the processor, the trained reinforcement learning agent to adjust the third set of weighted dose-volume objectives; and generating, by the processor, a third radiotherapy treatment plan for the third patient based on the third adjusted set of weighted dose-volume objectives.

In some cases, determining the reward value comprises determining, by the processor, the reward value based on a comparison of the difference between the third three-dimensional dose distribution and a second threshold.

In some cases, determining the reward value comprises applying, by the processor, a set of criteria to the third three-dimensional dose distribution; and determining, by the processor, the reward value based on the application of the set of criteria to the third three-dimensional dose distribution.

In some cases, determining the reward value comprises responsive to determining the third three-dimensional dose distribution is within the threshold of the second predicted three-dimensional dose distribution, applying, by the processor, a set of criteria to the third three-dimensional dose distribution; and determining, by the processor, the reward value based on the application of the set of criteria to the third three-dimensional dose distribution.

In some cases, generating the set of weighted dose-volume objectives of the cost function comprises assigning, by the processor, one or more weights according to a stored template of weights that indicates weights to apply to different structures of the patient.

In some cases, generating the set of weighted dose-volume objectives of the cost function comprises assigning, by the processor, one or more weights according to a stored ranked list of targets for the radiotherapy treatment plan.

In some cases, adjusting the set of weighted dose-volume objectives comprises inserting, by the processor using the reinforcement learning agent, one or more second objectives and corresponding weights into the set of weighted dose-volume objectives, the one or more second objectives corresponding to different structures of the patient.

In some cases, each objective of the set of weighted dose-volume objectives corresponds to a different structure within the patient and a different reinforcement learning agent of a plurality of reinforcement learning agents, and wherein adjusting the set of weighted dose-volume objectives of the cost function comprises adjusting, by the processor, the set of weighted dose-volume objectives using the plurality of reinforcement learning agents.

In an embodiment, a system comprises one or more processors coupled with memory, the memory comprising instructions that, when executed by the one or more processors, cause the one or more processors to execute a dose prediction machine learning model using one or more treatment attributes for a patient to generate a predicted three-dimensional dose distribution for the patient; generate a set of weighted dose-volume objectives of a cost function based on the predicted three-dimensional dose distribution for the patient; determine a first three-dimensional dose distribution that reduces a first cost value of the cost function; determine a difference between the first three-dimensional dose distribution and the predicted three-dimensional dose distribution; adjust, using a reinforcement learning agent, the set of weighted dose-volume objectives of the cost function based on the difference between the first three-dimensional dose distribution and the predicted three-dimensional dose distribution; determine a second three-dimensional dose distribution that reduces a second cost value based on the adjusted set of weighted dose-volume objectives of the cost function; and responsive to determining a difference between the first cost value and the second cost values satisfies a threshold, generate a radiotherapy treatment plan for the patient based on the adjusted set of weighted dose-volume objectives.

In some cases, the instructions further cause the one or more processors to execute the dose prediction machine learning model using one or more second treatment attributes for a second patient to generate a second predicted three-dimensional dose distribution for the second patient; generate a second set of weighted dose-volume objectives of a second cost function based on the second predicted three-dimensional dose distribution for the second patient; determine a third three-dimensional dose distribution that reduces a third cost value of the second cost function; determine a second difference between the third three-dimensional dose distribution and the second predicted three-dimensional dose distribution; determine a reward value at least according to the difference between the third three-dimensional dose distribution and the second predicted three-dimensional dose distribution; and train the reinforcement learning agent based on the reward value.

In some cases, the instructions further cause the one or more processors to receive third one or more treatment attributes of a third radiotherapy treatment plan for a third patient; generate a third set of weighted dose-volume objectives based on the third one or more treatment attributes for the third patient; execute the trained reinforcement learning agent to adjust the third set of weighted dose-volume objectives; and generate a third radiotherapy treatment plan for the third patient based on the third adjusted set of weighted dose-volume objectives.

In some cases, the instructions cause the one or more processors to determine the reward value by determining the reward value based on a comparison of the difference between the third three-dimensional dose distribution and a second threshold.

In some cases, the instructions cause the one or more processors to determine the reward value by applying a set of criteria to the third three-dimensional dose distribution; and determining the reward value based on the application of the set of criteria to the third three-dimensional dose distribution.

In some cases, the instructions cause the one or more processors to determine the reward value by, responsive to determining the third three-dimensional dose distribution is within the threshold of the second predicted three-dimensional dose distribution, applying a set of criteria to the third three-dimensional dose distribution; and determining the reward value based on the application of the set of criteria to the third three-dimensional dose distribution.

In some cases, the instructions cause the one or more processors to generate the set of weighted dose-volume objectives of the cost function by assigning one or more weights according to a stored template of weights that indicates weights to apply to different structures of the patient.

In some cases, the instructions cause the one or more processors to generate the set of weighted dose-volume objectives of the cost function by assigning one or more weights according to a stored a ranked list of targets for the radiotherapy treatment plan.

In some cases, the instructions cause the one or more processors to adjust the set of weighted dose-volume objectives by inserting, using the reinforcement learning agent, one or more second objectives and corresponding weights into the set of weighted dose-volume objectives, the one or more second objectives corresponding to different structures of the patient.

In some cases, each objective of the set of weighted dose-volume objectives corresponds to a different structure within the patient and a different reinforcement learning agent of a plurality of reinforcement learning agents, and wherein the instructions cause the one or more processors to adjust the set of weighted dose-volume objectives of the cost function by adjusting the set of weighted dose-volume objectives using the plurality of reinforcement learning agents.

## BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.

**FIG. 1** illustrates components of a reinforcement learning plan generation system, according to an embodiment.

**FIG. 2** illustrates a process flow diagram executed in a reinforcement learning plan generation system, according to an embodiment.

**FIG. 3** illustrates a sequence diagram illustrating operation of a reinforcement learning plan generation system, according to an embodiment.

**FIG. 4** illustrates a process flow diagram for training a reinforcement learning agent, according to an embodiment.

## DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting of the subject matter presented.

A computer model may use a cost function of objectives to determine radiation dose distributions between different structures (e.g., organs, bones, etc.) of a patient to generate a radiotherapy treatment plan for the patient. To do so, the computer may use a starting point of initial objectives and iteratively adjust the objectives using an optimization algorithm until determining an optimal dose distribution for the patient. The initial objectives can be originally input by a user. However, because the initial objectives may be input "blindly" without regard to the patient being treated, determining the optimal objectives for the radiotherapy treatment plan can require a large number of iterations of adjusting the objectives and determining dose distributions based on the adjusted objectives. Such iterative processing can require a large amount of time and processing resources.

For the aforementioned reasons, there is a desire for a computer model to generate treatment attributes starting with an initial set of objectives which are closer to expected achievable objectives or values. Starting with such an initial set of objectives can significantly reduce the number of processing repetitions that are needed to generate optimal objectives of a radiotherapy treatment plan. Additionally, there is a desire for an improved artificial intelligence modeling/training technique to train a model to generate optimal dose distributions with fewer iterations of processing and in a manner that is computationally efficient and cost-effective that produces timely results.

Using the methods and systems discussed herein, a processor can train and use a reinforcement learning model in combination with a dose prediction machine learning model to automatically generate a radiotherapy treatment plan. The processor can receive a set of treatment attributes for a patient. The treatment attributes can include any combination of one or more of computed tomography (CT) images, field geometry settings, dose prescriptions, etc. The processor can execute a dose prediction machine learning model (e.g., a neural network, random forest, a support vector machine, etc.) using the treatment attributes to generate a predicted three-dimensional dose distribution for the patient. The processor can use the predicted three-dimensional dose distribution to generate a cost function that includes a weighted set of objectives. The objectives can each correspond to a different structure of the patient. The processor can use an optimization algorithm to reduce (e.g., minimize) the cost function of the objectives to generate a first three-dimensional dose distribution for treating the patient.

The processor can use a reinforcement learning agent to compare the first three-dimensional dose distribution with the predicted three-dimensional dose distribution generated by the dose prediction machine learning model. The reinforcement learning agent can adjust objectives and/or weights of the cost function to bring the first three-dimensional dose distribution closer to (e.g., have closer values to) the predicted three-dimensional dose distribution based on the comparison. In some cases, the reinforcement learning agent can be trained to drive the optimization process to a better dose distribution than the predicted three-dimensional dose distribution. For example, during training, the reinforcement learning agent can receive positive rewards for improving organ at risk protection from levels indicated in the predicted three-dimensional dose distribution. In using the reinforcement learning agent trained in this way, the processor can generate an adjusted set of weighted dose-volume objectives of a cost function based on the predicted three-dimensional dose distribution for the patient. The processor can determine a second three-dimensional dose distribution that reduces (e.g., minimizes) a cost value of the adjusted cost function. The reinforcement learning agent can compare the sequentially determined cost values with each other to determine a difference. The reinforcement learning agent can compare the difference to a threshold. Responsive to determining the difference exceeds the threshold, the reinforcement learning agent can adjust the cost function for a second time. The processor can repeat the process of using the reinforcement learning model to iteratively adjust the objectives of the cost function based on differences between the predicted three-dimensional dose distribution and three-dimensional dose distributions generated based on the adjusted cost function until determining the cost value of the cost function converges (e.g., does not change above a threshold) between two or more iterations. In some cases, the reinforcement learning agent can continue adjusting the objectives and/or weights to optimize or improve other plan quality metrics (e.g., length of treatment, number of treatments, etc.). The reinforcement learning agent can adjust the objectives and/or weights until determining any number of metrics satisfy criteria or an internal policy of the reinforcement learning agent.

The processor can use the objectives of the final cost function in a radiotherapy treatment plan for the patient; for example the processor can use the objectives of the final cost function to generate a radiotherapy treatment plan for the patient. By using the predicted dose prediction generated by the dose prediction machine learning model based on treatment attributes of the patient in combination with a reinforcement learning agent, the processor can generate radiotherapy treatment plans using a starting point that is closer to the optimal set of objectives for the radiotherapy treatment plan for the patient and reach the optimal set of objectives faster (e.g., in fewer iterations). Thus, the processor can generate the radiotherapy treatment plan with less latency and using fewer processing resources. Additionally, by using predicted dose distributions generated based on attributes of individual patients as a starting point, the processor can generate radiotherapy treatment plans that are personalized to the individual patients.

Accordingly, the processor implementing the systems and methods described herein can automatically generate optimized radiotherapy treatment plans using a reinforcement learning model and a dose prediction machine learning model, reducing the number of iterations of generating and adjusting objectives of the radiotherapy treatment plans and the latency and processing resources that are required to do so.

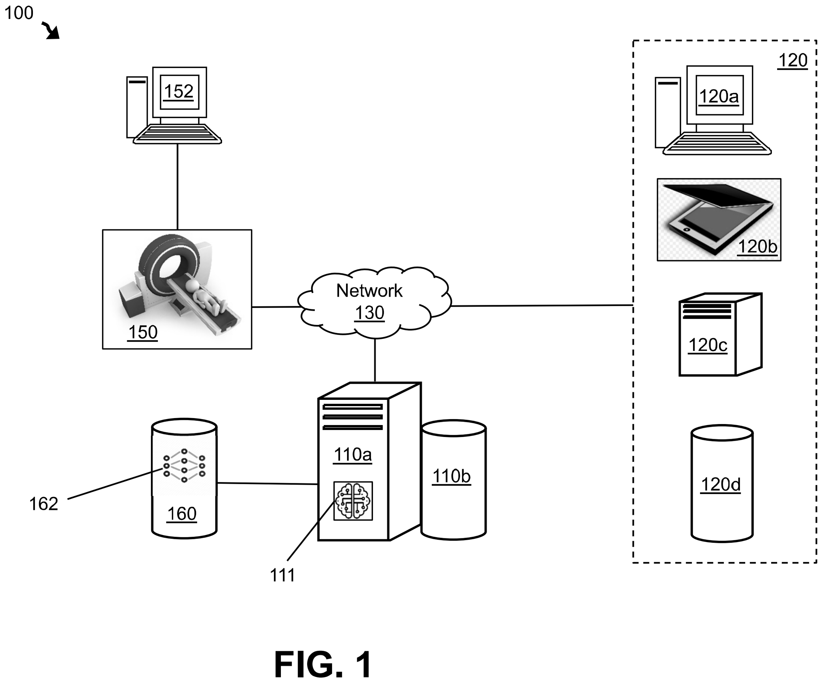

For example, **FIG. 1** illustrates components of a reinforcement learning plan generation system **100,** according to an embodiment. The system **100** may include an analytics server **110a,** a system database **110b,** a reinforcement learning agent **111,** end-user devices **120a-d** (collectively end-user devices **120),** a medical device **150,** a medical device computer **152,** a database **160,** and a dose prediction machine learning model **162.** Various components depicted in **FIG. 1** may belong to a radiation therapy treatment clinic at which patients may receive radiation therapy treatment, in some cases via one or more radiation therapy machines (e.g., the medical device **150).**

The system **100** is not confined to the components described herein and may include additional or other components, not shown for brevity, which are to be considered within the scope of the embodiments described herein.

The above-mentioned components may be connected to each other through a network **130.** Examples of the network **130** may include, but are not limited to, private or public local-area networks (LAN), wireless local-area networks (WLAN), metropolitan-area networks (MAN), wide-area networks (WAN), and the Internet. The network **130** may include wired and/or wireless communications according to one or more standards and/or via one or more transport mediums. The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), or EDGE (Enhanced Data for Global Evolution) network.

The analytics server **110a** may generate and display an electronic platform configured to interface a user with the reinforcement learning agent **111** and for receiving patient and/or treatment information or attributes and outputting the results of execution of the reinforcement learning agent **111** and/or the dose prediction machine learning model **162.** The electronic platform may include graphical user interfaces (GUI) displayed on each of the end-user devices **120,** the medical device **150,** and/or the medical device computer **152.** An example of the electronic platform generated and hosted by the analytics server **110a** may be a web-based application or a website configured to be displayed on different electronic devices, such as mobile devices, tablets, personal computers, and the like.

The information displayed by the electronic platform can include, for example, input elements to receive data associated with a patient to be treated (e.g., plan objectives or targets) and display results of predictions produced by the reinforcement learning agent **111** (e.g., text, image, or video generated in response to inputs received through the electronic platform). The analytics server **110a** may then display the results for a medical professional and/or directly revise one or more operational attributes of the medical device **150.** In some embodiments, the medical device **150** can be a diagnostic imaging device or a treatment delivery device.

The analytics server **110a** may be any computing device comprising a processor and non-transitory machine-readable storage capable of executing the various tasks and processes described herein. The analytics server **110a** may employ various processors such as central processing units (CPU) and graphics processing unit (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

End-user devices **120** may be any computing device comprising a processor and a non-transitory machine-readable storage medium capable of performing the various tasks and processes described herein. Non-limiting examples of an end-user device **120** may be a workstation computer, laptop computer, tablet computer, and server computer. In operation, various users may use end-user devices **120** to access the GUI operationally managed by the analytics server **110a.** Specifically, the end-user devices **120** may include clinic computer **120a,** clinic server **120b,** and a medical professional device **120c.** Even though referred to herein as "end-user" devices, these devices may not always be operated by end-users. For instance, the clinic server **120b** may not be directly used by an end user. However, the results stored onto the clinic server **120b** may be used to populate various GUIs accessed by an end user via the medical professional device **120c.**

The medical device **150** may be a radiation therapy machine configured to implement a patient's radiation therapy treatment. The medical device **150** may also be in communication with a medical device computer **152** that is configured to display various GUIs discussed herein. For instance, the analytics server **110a** may display the results generated by the reinforcement learning agent **111** onto the computing devices described herein. In a non-limiting example, the GUI may display objectives of a cost function and/or a three-dimensional dose distribution of a radiotherapy treatment plan generated by the reinforcement learning agent **111** at the medical device computer **152** and/or the end-user devices **120.**

The dose prediction machine learning model **162** may be stored in the database **160.** The database **160** and the database **110b** may be the same or different databases. The dose prediction machine learning model **162** may be or include a machine learning model (e.g., a neural network, a support vector machine, a random forest, etc.) that is configured to process treatment attributes for patients to generate predicted three-dimensional dose distributions for the patients. In some cases, the dose prediction machine learning model **162** can be or include a linear regression model that predicts two-dimensional dose information, such as in the form of dose-volume histogram (DVH) curves. Treatment attributes can be or include any type of patient attribute, such as, for example, any combination of one or more of: height, gender, weight, treatment options for the patient, machine attributes (e.g., gantry movements, gantry positions, etc.), treatment objectives, attributes regarding a tumor (e.g., size or shape), images of the patient or tumor, tumor stage, the primary site of treatment, endpoints, whether the tumor has been extended, body mass index, blood pressure, medical history (e.g., previous medical treatments received by the patient), etc. The dose prediction machine learning model **162** may be trained using supervised, semi-supervised, or unsupervised training methods to generate predicted three-dimensional dose distributions for patients based on such treatment attributes.

For example, the analytics server **110a** can train the dose prediction machine learning model by inputting a set of treatment attributes into the dose prediction machine learning model **162.** The analytics server **110a** can execute the dose prediction machine learning model **162** based on the input to generate a predicted three-dimensional dose distribution. The analytics server **110a** can compare the predicted three-dimensional dose distribution with an expected or ground truth three-dimensional dose distribution to determine a difference, such as by using a loss function. The analytics server **110a** can train the dose prediction machine learning model **162** based on the difference using back-propagation techniques, such as by adjusting internal weights and/or parameters e.g. of the dose prediction machine learning model, based on the difference. The analytics server **110a** can repeat this process any number of times until determining the dose prediction machine learning model **162** is accurate to a threshold (e.g., an accuracy threshold). In some cases, the analytics server **110a** can implement testing and validation stages before deploying the dose prediction machine learning model **162.** Analytics server **110a** can deploy (e.g., begin using) the dose prediction machine learning model **162** to generate predicted three-dimensional dose distribution responsive to determining the dose prediction machine learning model **162** is accurate to the threshold.

The reinforcement learning agent **111** may be stored in the system database **110b.** The reinforcement learning agent **111** may be configured or trained to make decisions and take actions within an environment to generate optimal three-dimensional dose distributions for radiotherapy treatment plans for patients, such as by maximizing a cumulative reward value. The reinforcement learning agent **111** can be trained to operate on a framework of states and/or to iteratively adjust objectives and/or weights of a cost function generated from objectives of radiotherapy treatment plans.

The analytics server **110a** can train the reinforcement learning agent **111.** To do so, the analytics server **110a** can identify or calculate reward values to train the reinforcement learning agent **111** based on respective differences between three-dimensional dose distributions that minimize cost values of the cost functions and predicted three-dimensional dose distributions generated by the dose prediction machine learning model **162.** The reinforcement learning agent **111** can update an internal policy (e.g., internal weights and/or parameters) and adjust the weights and/or objectives of the cost functions based on the respective reward values. Through iterative interactions, the agent can use algorithms such as Q-learning or policy gradients for training, improving the reinforcement learning agent **111** decision-making capabilities over time to generate objectives of radiotherapy treatment plans for different patients faster and with fewer iterations of adjusting the objectives and/or weights of cost functions. By leveraging exploration and exploitation strategies in this way, the reinforcement learning agent **111** can efficiently learn to generate optimal objectives for radiotherapy treatment plans using fewer and fewer iterations of adjusting cost functions initially generated from a predicted three-dimensional dose distribution output by the dose prediction machine learning model **162** from personalized treatment attributes of patients undergoing radiotherapy treatment.

In one example of training the reinforcement learning agent **111,** the analytics server **110a** can determine a reward value based on a difference between a predicted three-dimensional dose distribution and a three-dimensional dose distribution that reduces a cost function. The analytics server **110a** can determine the reward value to be proportional to the difference (e.g., the higher the difference the higher the reward value or the lower the difference the lower the reward value). In one example, the direction of the difference can be taken into account during training. For instance, a goal for a radiotherapy treatment plan may to achieve a certain organ at risk sparing level (e.g., a mean dose of 15Gy). Accordingly, if the current optimized dose is much higher, the reward value may be negative or a penalty. However, if the current optimized dose is much smaller than 15Gy, the reward value may be positive or a reward. The reinforcement learning agent **111** can determine reward values using such rules and/or as a distance (e.g., a weighted distance) measure between a predicted three-dimensional dose distribution and an optimized three-dimensional dose distribution, in some cases with different weights for different regions or structures of the body. The weights can correspond to the uncertainty levels of the dose prediction model in generating the predicted three-dimensional dose distribution. For example, the reinforcement learning agent may adjust the weighted dose-volume objectives of the cost function based on reward values; the reinforcement learning agent may determine reward values using a weighted distance measure between the first three-dimensional dose distribution and the predicted three-dimensional dose distribution, wherein the weights may be for different regions or structures of the body, and wherein the weights may correspond to the uncertainty levels of the dose prediction model in generating the predicted three-dimensional dose distribution.

During training, the reinforcement learning agent **111** can adjust the objectives and/or the weights of the cost function to maximize the reward value, such as by making it more likely that the cost function corresponds to a three-dimensional dose distribution with a reduced (e.g., minimized) cost value that is closer to the predicted three-dimensional dose distribution.

The analytics server **110a** can train the reinforcement learning agent **111** to drive a radiotherapy treatment plan optimization to better dose distributions than predicted dose distributions generated by the dose prediction machine learning model **162**. The analytics server **110a** can do so, for example, using a step-based reward value determination in which an analytics server **110a** initially determines rewards based on a difference or distance between a predicted three-dimensional dose distribution and an optimized three-dimensional dose distribution across iterations until determining such a different or distance is below a threshold. Responsive to determining the difference or distance is below the threshold, the analytics server **110a** can use one or more rules of a set of criteria (e.g., a set of criteria that are satisfied if different aspects of a three-dimensional dose distribution are improvements over a predicted three-dimensional dose distribution) to adjust (e.g., increase or decrease) the reward value. Accordingly, the reinforcement learning agent can initially generate reward values towards generating a three-dimensional dose distribution which resembles the predicted three-dimensional dose distribution. After that, the reinforcement learning agent can generate reward values that may be smaller, but still positive, if the reinforcement learning agent can generate a more effective three-dimensional dose distribution than the predicted three-dimensional dose distribution.

The analytics server **110a** can implement the reinforcement learning agent **111** after training the reinforcement learning agent **111**. For example, the analytics server **110a** can receive a request to generate a radiotherapy treatment plan for a patient from an end-user device **120**. The analytics server **110a** can receive the request with an identifier of the patient. In some cases, the request can include one or more treatment attributes for the patient. In some cases, the analytics server can use the identifier of the patient to query the database **110b** to identify treatment attributes for the patient. The analytics server **110a** can receive, identify, or otherwise obtain treatment attributes for the patient in any way and use the treatment attributes to generate a radiotherapy treatment plan (e.g., responsive to the request).

The analytics server **110a** can use the treatment attributes for the patient to generate objectives including treatment objectives for treating the patient. The treatment objectives can correspond to an amount of radiation (e.g., dosage) to use to treat different structures of the patient. The analytics server **110a** can execute the dose prediction machine learning model using the treatment attributes for the patient (e.g., using the treatment attributes for the patients as input). The execution can cause the dose prediction machine learning model **162** to generate a predicted three-dimensional dose distribution for treating the patient. The analytics server **110a** can generate objectives for treating the patient from the predicted three-dimensional dose distribution. The analytics server **110a** can weight the different objectives in a cost function based on a template of stored weights that correspond to the different objectives (e.g., structures) or based on a stored ranking of the different objectives and/or structures corresponding to the objectives. The weighted set of objectives can make up the cost function generated from the predicted three-dimensional dose distribution.

The analytics server **110a** can implement the reinforcement learning agent **111** to adjust the cost function. First, the analytics server **110a** can use an optimization algorithm (e.g., gradient descent, gradient-based optimization method, model-based optimization, etc.) on a cost function containing the objectives to generate a three-dimensional dose distribution that minimizes the cost function (e.g., minimizes or reduces a cost or cost value of the cost function). Then, the reinforcement learning agent **111** can adjust the cost function according to a difference between the generated three-dimensional dose distribution and the predicted three-dimensional dose distribution output by the dose distribution machine learning model. For example, the analytics server **110a** can use an optimization algorithm on the cost function to generate a three-dimensional dose distribution that reduces (e.g., minimizes) a cost value of the cost function. The reinforcement learning agent **111** can compare the generated three-dimensional dose distribution to the predicted three-dimensional dose distribution generated by the dose prediction machine learning model to determine a difference between the generated three-dimensional dose distribution and the predicted three-dimensional dose distribution. The reinforcement learning agent **111** can adjust the objectives and/or the weights of the cost function according to a learned policy of the reinforcement learning agent **111,** such as by making it more likely that the cost function corresponds to a three-dimensional dose distribution with a reduced (e.g., minimized) cost that is closer to the predicted three-dimensional dose distribution. The analytics server **110a** can use the optimization algorithm on the cost function to generate another three-dimensional dose distribution that reduces (e.g., minimizes) a cost value of the cost function. The analytics server **110a** can repeat this process any number of times, comparing sequentially generated cost values for each iteration. The analytics server **110a** can compare the changes or differences between cost values to a threshold. The analytics server **110a** can stop repeating the process responsive to determining a change or difference between cost values is lower than the threshold or the cost value has otherwise converged.

In some cases, the reinforcement learning agent **111** can additionally determine whether other criteria are met before determining the objectives are finalized or the objectives have converged. For example, the reinforcement learning agent **111** can determined the mean dose levels delivered to defined organs at risk are below a threshold and/or determine whether target coverage metrics satisfy defined objectives. If, after several iterations, the reinforcement learning agent **111** determines that there is no improvement in these metrics, the reinforcement learning agent **111** can determine that the cost function has converged (e.g., instead of or in addition to determining the objectives of the cost function has converged).

The analytics server **110a** can generate a radiotherapy treatment plan based on the adjusted objectives of the cost function. For example, the analytics server **110a** can identify the objectives of the cost function after the reinforcement learning agent **111** stops iteratively updating the cost function (e.g., upon determining a change or difference between cost values is lower than the threshold or the cost value has otherwise converged). The analytics server **110a** can insert the objectives into a record containing the radiotherapy treatment plan (e.g., the objectives and/or any other treatment attributes of the radiotherapy treatment plan). In doing so, the analytics server **110a** can include the objectives of the cost function in the radiotherapy treatment plan for the patient. In doing so, the reinforcement learning agent can improve upon the initial predicted three-dimensional dose distribution.

The analytics server **110a** can present the radiotherapy treatment plan including the objectives as adjusted by the reinforcement learning agent **111** on a user interface of an end-user device **120** (e.g., the same end-user device **120** that requested the radiotherapy treatment plan for the patient). A user (e.g., the patient or a medical professional treating the patient) accessing the end-user device **120** can view the radiotherapy treatment plan and/or implement the radiotherapy treatment plan or the analytics server, or the end-user device **120** can use the radiotherapy treatment plan to control (e.g., automatically control) the medical device **150** to treat the patient. In some cases, the analytics server **110a** can transmit the radiotherapy treatment plan to the medical device **150** and a controller of the medical device **150** can use the radiotherapy treatment plan to automatically control the medical device **150** to treat the patient.

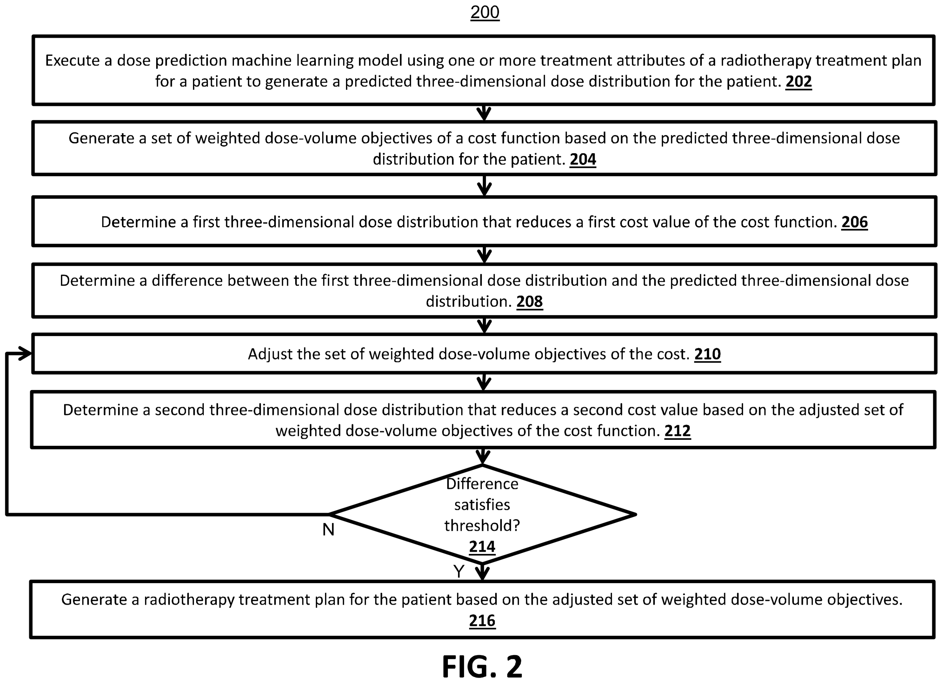

Referring now to **FIG. 2,** a method **200** shows an operational workflow executed in a reinforcement learning plan generation system, in accordance with an embodiment. The method **200** may include steps **202-216.** However, other embodiments may include additional or alternative steps or may omit one or more steps altogether. The method **200** is described as being executed by a server, such as the analytics server described in **FIG. 1.** However, one or more steps of the method **200** may be executed by any number of computing devices operating in the distributed computing system described in **FIG. 1.** For instance, one or more computing devices may locally perform part or all of the steps described in **FIG. 2.**

Using the method **200,** the analytics server can implement a combination of a dose prediction machine learning model and a reinforcement learning agent to generate a radiotherapy treatment plan. To do so, the analytics server can execute a dose prediction machine learning model using treatment attributes of a patient to generate a predicted three-dimensional dose distribution for treating the patient. The analytics server can generate objectives (e.g., dose-volume objectives) from the predicted three-dimensional dose distribution that correspond to different structures within the patient. The analytics server can assign weights to the objectives. The analytics server can use an optimization algorithm on a cost function (e.g., apply weights of a cost function) to the objectives to determine a three-dimensional dose distribution that reduces (e.g., minimizes) a cost value of the cost function. The analytics server can use a reinforcement learning agent to compare the three-dimensional dose distribution with the predicted three-dimensional dose distribution to determine a difference between the two distributions. The reinforcement learning agent can adjust one or more objectives and/or weights of the cost function based on the difference. The analytics server can apply the cost function (e.g., the adjusted weights of the cost function with any operations of the cost function) to the adjusted objectives and iteratively repeat the process until the cost value converges (e.g., until the reinforcement learning agent determines changes or differences between sequence cost values are below a threshold). The analytics server can use the final objectives in a radiotherapy treatment plan for the patient.

For example, at step **202,** the analytics server can execute a dose prediction machine learning model. The analytics server can execute the dose prediction machine learning model using treatment attributes for a patient (e.g., using treatment attributes for the patient as input). Treatment attributes can be or include, for example, one or more of any type of patient attribute, such as, for example, height, gender, weight, treatment options for the patient, machine attributes (e.g., gantry movements, gantry positions, etc.), treatment objectives, attributes regarding a tumor (e.g., size or shape), images of the patient or tumor, tumor stage, the primary site of treatment, endpoints, whether the tumor has been extended, body mass index, blood pressure, medical history (e.g., previous medical treatments received by the patient), etc. The dose prediction machine learning model can be a machine learning model (e.g., a neural network, a random forest, a support vector machine, etc.) configured to process treatment attributes to generate three-dimensional dose predictions for treating patients corresponding to the respective treatment attributes.

The analytics server can input the treatment attributes for the patient and execute the dose prediction machine learning model to cause the dose prediction machine learning model to generate a predicted three-dimensional dose distribution. A three-dimensional dose distribution can be or include a set of voxels in a three-dimensional grid that represents different portions of a patient. Each voxel (e.g., volume element) can correspond to a dose value in the three-dimensional dose distribution. In some cases, the analytics server can input the treatment attributes for the patient in response to a request from a user or another computing device to generate a radiotherapy treatment plan for the patient.

At step **204,** the analytics server can generate a set of weighted dose-volume objectives of a cost function. The analytics server can do so using the predicted three-dimensional dose distribution and structure contours that the analytics server receives or identifies for the patient. The analytics server can identify regions of interest within the three-dimensional dose distribution. Regions of interest can be or correspond to structures of the patient, such as organs, tumors, and/or any other anatomical structures (e.g., structures relevant to the treatment, such as organs, tumors, and/or any target structures for treatment). The analytics server can identify voxels of the predicted three-dimensional dose distribution that correspond to the regions of interest and extract or identify the dose values from the identified voxels. In doing so, the analytics server can create or generate a list of dose values for each region of interest. The analytics server can sort the extracted dose values in ascending order. The analytics server can bin these dose values into discrete dose bins. The bin width can vary, but a few examples of bin widths can include 0.1 Gy or 0.5 Gy increments. The analytics server can determine the volume of the region of interest that falls within each dose bin. The analytics server can do so by counting the number of voxels in each dose bin and multiplying by the volume of a single voxel.

The analytics server can create one or more dose-volume histogram (DVH) curves using the dose bins. For example, for cumulative DVH (cDVH), the analytics server can sum the volumes from the highest dose bin down to the lowest. In doing so, the analytics server can generate the cumulative volume receiving at least a given dose. In another example, for differential DVH (dDVH), the analytics server can use the volumes calculated in each dose bin directly. The analytics server can normalize the DVH. The analytics server can normalize the volume data to either the total volume of the region of interest (e.g., to obtain a relative volume, typically expressed as a percentage) or to the volume of each voxel (e.g., if dealing with absolute volumes). The analytics server can plot a DVH curve. The analytics server can plot the dose (x-axis) against the normalized cumulative volume (y-axis) for a cDVH or against the volume per bin for a dDVH.

The analytics server can use the DVH curves or otherwise the DVH to create objectives including one or more objectives for the individual structures of the patient. To do so, the analytics server can identify clinical goals for treating the patient. The clinical goals can identify the percentages of individual structures (e.g., target tumor and/or organs at risk) that are targeted to receive less than or greater than a specified dose, for example. The analytics server can identify the clinical goals from a user input and/or from the treatment attributes that the analytics server obtains from a database or the request for a radiotherapy treatment plan. For each region of interest (e.g., tumor and/or organ at risk), the analytics server can generate objectives that represent the DVH curves. Objectives may include, for example, maximum dose, minimum dose, mean dose, and/or volume constraints.

The analytics server can assign weights of a cost function to the objectives. The analytics server can assign the weights to the objectives using a stored template. For example, the analytics server can store one or more stored templates that each correspond to a set of treatment attributes. The set of treatment attributes can include, for example, a location of a tumor, a size of the tumor, patient demographics, and/or any permutation or combination of treatment attributes. Each stored template can include a weight (e.g., a defined weight) for individual objectives that may be used in a cost function that can be used to generate a radiotherapy treatment plan. The analytics server can retrieve a template that corresponds to a set of treatment attributes that match (e.g., exactly match or match a number of treatment attributes above a threshold) the treatment attributes obtained for generating the radiotherapy treatment plan for the patient.

In another example, the analytics server can assign weights to the objectives according to a ranked list of targets (e.g., clinical goal, for example a ranked list of clinical goals) for the individual objectives. For example, the analytics server can assign higher weights to objectives that are associated with (e.g., mapped to in memory) high-ranked clinical goals than objectives that are associated with lower ranked clinical goals. In cases in which multiple objectives correspond to the same structure or target, the analytics server can assign the same or identical weights to the objectives. The ranking of the targets can be input or correspond to stored templates that correspond to sets of treatment attributes similar to the above. The objectives and corresponding weights can make up a cost function.

The analytics server can use one or more types of objectives in a cost function to generate the radiotherapy treatment plan. For example, the cost function can include lower objectives (e.g., lower dose limits), upper objectives (e.g., upper dose limits), cubic objectives, upper line objectives, and/or generalized equivalent uniform dose (gEUD) objectives. The cost function can include a weight for each of one or more of the objectives. The lower objective can include three parameters: target dose level $D_{target}$ at DVH chart volume position $V$, and priority p. If a structure's DVH line falls short from point ($D_{target}$, $V$) in the DVH chart, all points x in the structure that have dose $D(x) < D$ and contribute to the DVH line under volume $V$ cause cost using a square law:

$$\text{Cost}\left(D(x)\right) = \text{weight} * \left(D_{target} - D(x)\right)^2$$

Structures (e.g., all structures) that have lower objectives can be considered as target structures for radiation. Arc optimization for lower objectives can use a special type of calculation if it has volume value of 100%, such as the calculations for cubic objectives described below.

Upper objectives can have three parameters: target dose level $D_{target}$ at DVH chart volume position $V$, and priority $p$. If the structure's DVH line goes above of point ($D_{target}$, $V$) in DVH chart, all voxels $x$ in the structure that have dose $D(x) > D$ and contribute to the DVH line over volume $V$ cause cost using square law:

$$\text{Cost}\left(D(x)\right) = \text{weight} * \left(D_{target} - D(x)\right)^2$$

Arc optimization for upper objectives can use a special type of calculation if it has volume value of 0%, such as the calculations for cubic objectives described below.

Cubic objectives can be used in volumetric modulated arc therapy (VMAT) optimization with the same objectives as intensity-modulated radiation therapy (IMRT) optimization to identify pronounced cold and hot spots due to the continuous movement of gantry and less MU's per gantry angle. All lower objectives at 100% volume and upper objectives at 0% volume can be handled such that a dose limit $D_{spike}$, defining what is cold/hot for this objective, is re-adjusted at each iteration. The cost is normalized to be continuous at the starting parametrization with parameter $h$. The cost function is then calculated as (for upper objectives, lower objectives use the same logic):

$$\text{Cost}\left(D(x)\right) = \text{weight} * \left(D_{target} - D(x)\right)^2, D(x) \leq D_{spike}$$

$$\text{Cost}\left(D(x)\right) = \text{weight} * h * \left(D_{target} - D(x)\right)^3, D(x) > D_{spike}$$

Upper line objectives can define target dose levels $D_{target}$ for the DVH chart volume positions $v$ with a common priority $p$. This target dose-volume curve can be constructed into a spline $D(v)$. If the structure's DVH line goes above any point in the spline, all voxels $x$ in the structure that have dose $D(x) > D(v)$ and contribute to the DVH line over volume $v$, cause cost according to square law:

$$\text{Cost}\left(D(x)\right) = \text{weight} * \left(D_{target} - D(x)\right)^2$$

Optimization can support three objective types for generalized Equivalent Uniform Dose (gEUD) value: lower gEUD, upper gEUD and exact (target) gEUD. Implementation of the objectives follows the formalism described in ref (7). gEUD value for volume (V) having dose D(x) and using parameter (a) can be calculated as:

$$\mathrm{gEUD}\left(a, V, D\right) = \left(\frac{1}{V} * \sum_{V} v(x)D(x)^a\right)^{\frac{1}{a}}$$

The cost functions described herein are only examples of cost functions. Any type of cost function, such as an exponential cost function, can be used when implementing the systems and methods described herein.

At step **206,** the analytics server can determine a first three-dimensional dose distribution that reduces (e.g., minimizes) a first cost value of the cost function. The analytics server can do so using a plan optimization algorithm. For example, the analytics server can use an optimization algorithm on a cost function containing the objectives that the analytics server generated for the patient using the dose prediction machine learning model. The cost function can apply penalties based on one or more rules when generating the first three-dimensional dose distribution using the cost function. For example, the rules can include penalizing doses that exceed a defined maximum dosage, penalizing deviations from a defined mean dose, and/or penalizing deviations from defined volume constraints at specified dose levels. In some cases, the defined values of the rules can be from the predicted three-dimensional dose distribution. In some cases, the defined values can be input or otherwise determined in another manner. The reinforcement learning agent can iteratively adjust the weights and/or parameters of the cost function until generating a three-dimensional dose distribution (e.g., that corresponds to the objectives, or control point parameters) that reduces or minimizes a first cost value of the cost function.

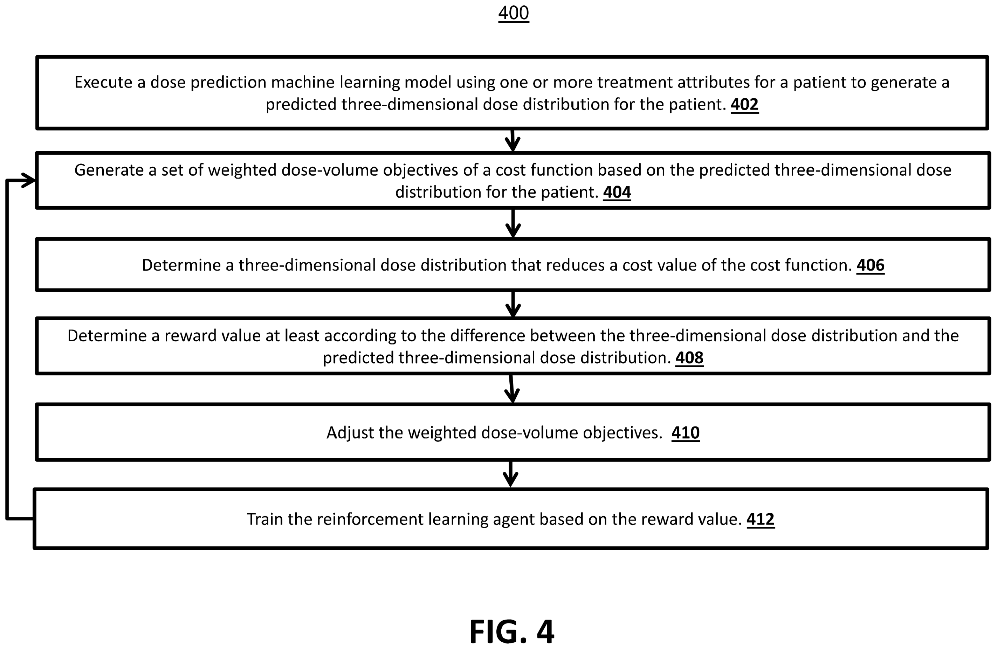

At step **208,** the analytics server can determine a difference between the first three-dimensional dose distribution and the predicted three-dimensional dose distribution. The analytics server can do so using a reinforcement learning agent. The reinforcement learning agent may be trained to adjust objectives and/or weights of cost functions according to an internal policy learned as described at least with reference to **FIG. 4.** For example, the reinforcement learning agent can compare the individual dosage values of the first three-dimensional dose distribution and the predicted three-dimensional dose distribution that correspond to the same structures or spaces of the patient. The reinforcement learning agent can use a function, such as a distance function, to determine a difference or distance between the two three-dimensional dose distributions. In some cases, the distance function may be weighted based on the structures corresponding to the dosage of the dose distributions. The weights can be pre-defined or can be proportional or otherwise based on a confidence (e.g., a probability or confidence score, such as out of 100) that the dose prediction machine learning model had in dosage in the predicted three-dimensional dose distribution for the different structures. The reinforcement learning agent can determine an adjustment (e.g., to one or more objectives and/or weights of the cost function) based on and/or proportional to the difference or distance.

In some cases, the reinforcement learning agent can use other factors to determine an adjustment to the weights and/or objectives of the cost function in addition to or instead of the difference between the predicted three-dimensional dose distribution and the first three-dimensional dose distribution. For example, the reinforcement learning agent can use a set of rules to determine the adjustment. For instance, the reinforcement learning agent can determine the adjustment based on whether individual structures receive at least a target dosage, are above a target dosage, or are within a range of a target dosage in the first three-dimensional dose distribution. In another example, the reinforcement learning agent can determine the adjustment based on whether structures labeled as not to receive any dosage, such as organs at risk (e.g., organs at risk that are far away from a target structure), receive any dosage. The reinforcement learning agent can determine the adjustment as a function of or otherwise based on any number of such determinations and/or using a learned or trained policy of the reinforcement learning agent.

At step **210,** the analytics server can adjust the set of weighted dose-volume objectives. The analytics server can use the reinforcement learning agent to do so. The reinforcement learning agent can adjust the set of weighted dose-values based on the adjustment determined at step **208.** For example, the reinforcement learning agent can adjust the set of weighted dose-volume objectives by adjusting the objectives and/or the weights associated with the objectives. In cases in which the weights were assigned according to a ranking system, the reinforcement learning agent may adjust the weights while preserving the relative order of the objective weights.

In some cases, the reinforcement learning agent may apply the learned policy of the reinforcement learning agent and add objectives for one or more structures to the cost function. The reinforcement learning agent can include weights for the added objectives, such as by maintaining a ranked order of structures, following a stored template, and/or according to any other rules. By adding structures to the cost function, the reinforcement learning agent can better control the dose level in certain spatial regions of the patient.

In some cases, the reinforcement learning agent determine adjustments to the objectives and/or weights that are proportional or otherwise based on the difference between the predicted three-dimensional dose distribution and the first three-dimensional dose distribution. For example, the reinforcement learning agent may make larger changes to the weighted objectives, such as by generating a new structure or by making larger changes in value when the difference value is lower. The reinforcement learning agent can make smaller changes to the weighted objectives when the difference is higher. By doing so, the reinforcement learning agent may reduce the number of iterations that are required to identify a set of weighted objectives that corresponds to a three-dimensional dose distribution for treating the patient.

At step **212,** the analytics server can determine a second three-dimensional dose distribution. The analytics server can determine the second three-dimensional dose distribution that reduces (e.g., minimizes) a second cost value of the cost function based on the adjusted set of weighted dose-volume objectives of the cost function. The analytics server can determine the second three-dimensional dose distribution that minimizes the second cost value in a similar manner to how the analytics server determined the first three-dimensional dose distribution that minimizes the first cost value at step **206.**

At step **214,** the analytics server can determine whether a difference between the first cost value and the second cost value satisfies a threshold (e.g., a cost threshold or second threshold). The analytics server can do so using the reinforcement learning agent and/or by comparing the difference to the threshold and determining whether the difference is less than the threshold. The data processing system can determine the difference satisfies the threshold responsive to determining the difference is less than the threshold, for example. Responsive to determining the difference exceeds the threshold, the reinforcement learning agent can iteratively repeat steps **210-214.**

In some cases, the reinforcement learning agent can determine whether the difference satisfies the threshold by determining whether the cost values are converging. The reinforcement learning agent can do so based on one comparison of a difference between cost values to a threshold or by iteratively repeating steps **210-214** and determining at least a threshold number of instances (e.g., sequential instances) in which differences between sequentially determined cost values satisfy the threshold.

In some cases, the analytics server can use multiple reinforcement learning agents when performing the steps **206-214.** For example, the analytics server can store separate reinforcement learning agents that are respectively trained or otherwise respectively correspond to adjusting individual objectives or objectives for individual structures. The reinforcement learning agents can be trained together in such a way that they can together control the overall plan generation process. The reinforcement learning agents that correspond to the same structure group (e.g., an organ at risk or target (e.g., planning volume)) can collaborate to achieve the common goals of the group, while the reinforcement learning agents that correspond to opposing structure groups can compete to identify the optimum trade-offs, such as by sparing the structures as much as possible in the organs at risk group or by providing better target coverage in the target group. Each reinforcement learning agent can be trained while generating the objectives for the radiotherapy treatment plan such that the reinforcement learning agents can more accurately and/or quickly generate objectives for radiotherapy treatment plans for future patients.

Responsive to determining the difference satisfies the threshold, at step **216,** the analytics server can generate a radiotherapy treatment plan for the patient. The analytics server can generate the radiotherapy treatment plan for the patient based on the adjusted set of weighted dose-volume objectives. For instance, the analytics server can identify the objectives of the adjusted set of weighted dose-volume objectives and include the objectives in the radiotherapy treatment plan. The analytics server can determine treatment attributes (e.g., gantry positioning settings) to reach the objectives and/or any other treatment attributes and include the determined treatment attributes in the radiotherapy treatment plan. The analytics server can include the three-dimensional dose distribution (e.g., the second three-dimensional dose distribution) that reduces the cost value of the adjusted set of weighted dose-volume objectives in the radiotherapy treatment plan. The objectives can be treatment attributes. The analytics server can store the radiotherapy treatment plan in memory, such as in a record (e.g., a file, document, table, listing, message, notification, etc.) containing an identifier of the patient in a database of memory.

The analytics server can present the radiotherapy treatment plan (e.g., the one or more treatment attributes, including the determined objectives, of the radiotherapy treatment plan) on a user interface presented at an end-user device. In some cases, the analytics server can present the radiotherapy treatment plan on the computer device that initially transmitted the request for the radiotherapy treatment plan for the patient.

In some embodiments, the analytics server can transmit the one or more treatment attributes of the radiotherapy treatment plan to a radiotherapy treatment machine to use to provide treatment to the patient. The radiotherapy treatment machine can operate (e.g., automatically operate) based on the one or more treatment attributes, such as to provide the dosage identified by the objectives of the radiotherapy treatment plan.

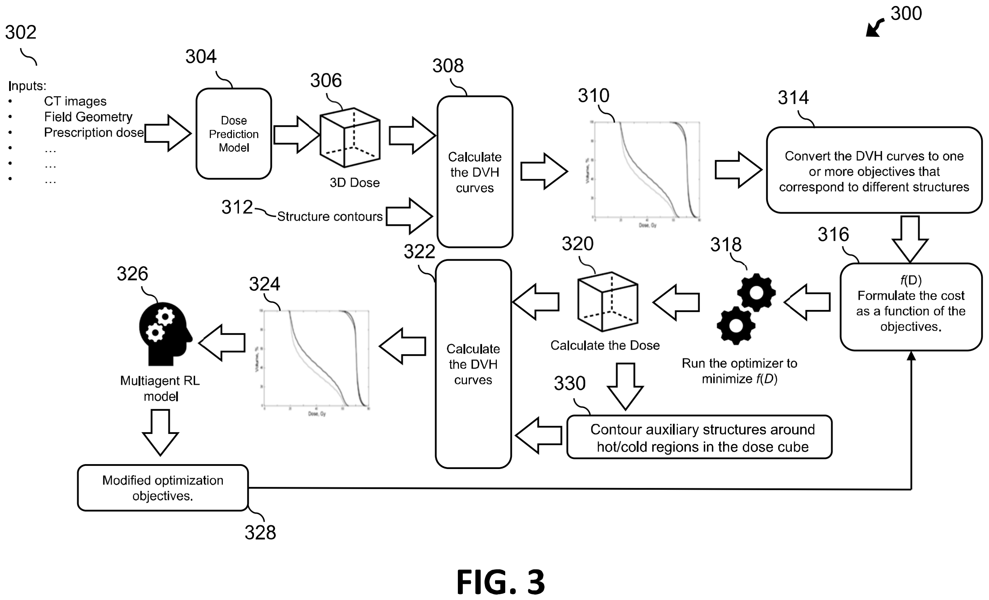

**FIG. 3** illustrates a sequence diagram illustrating a sequence **300** of operation of an already trained reinforcement learning plan generation system, according to an embodiment. Operations of the sequence **300** can be performed by any computing device or multiple computing devices. In one example, one or more of the operations of the sequence **300** can be performed by the analytics server **110a,** shown and described with reference to **FIG. 1.** Performance of the sequence **300** can facilitate the analytics server **110a** generating radiotherapy treatment plans using less processing power and with less latency over time.

In the sequence **300,** the analytics server **110a** can identify treatment attributes **302** (e.g., CT images, field geometry settings, prescription dosages, etc.) of a patient undergoing radiotherapy treatment. The analytics server **110a** can identify the treatment attributes from memory and/or in a request to generate a radiotherapy treatment plan. The analytics server **110a** can input the treatment attributes into a dose prediction machine learning model **304** that is trained to generate predictions of three-dimensional dose distributions based on input treatment attributes of individual patients. The analytics server **110a** can execute the dose prediction machine learning model **304** based on the input treatment attributes **302** to cause the dose prediction machine learning model **304** to generate a predicted three-dimensional dose distribution **306**. The analytics server **110a** can determine **308** one or more DVH curves **310** using the predicted three-dimensional dose distribution **306** and structure contours **312** of the patient. The analytics server **110a** can convert **314** the DVH curves **310** to one or more objectives that correspond to different structures (e.g., organs at risk and/or a target organ or tumor) of the patient.

The analytics server **110a** can formulate **316** or determine a cost function based on the objectives. The cost function can include one or more rules that apply penalties when the rules are not followed by three-dimensional dose distributions that are generated using the cost function. The cost function can include one or more weights for the objectives. The analytics server **110a** can execute **318** an optimizer (e.g., an optimization algorithm) to reduce (e.g., minimize) the cost function, which can include generating a new three-dimensional dose distribution **320** using the cost function and determining a cost value (e.g., a first cost value) for the three-dimensional dose distribution until reaching a minimum (e.g., a global or local minimum), for example. The analytics server **110a** can determine **322** one or more DVH curves **324** using the three-dimensional dose distribution **320** and/or structure contours **312** of the patient. The analytics server **110a** can execute one or more reinforcement learning agents **326** to determine a difference between the three-dimensional dose distribution **320** and the predicted three-dimensional dose distribution **306** (e.g., a difference between the DVH curves **324** and the DVH curves **310**). The reinforcement learning agents **326** can adjust **328** the weights and/or objectives of the cost function, which may include increasing or decreasing the weights and/or objectives, and/or adding one or more new objectives for additional or auxiliary structures of the patient, and/or removing one or more weights and/or objectives. The reinforcement learning agents **326** may do so based on the reinforcement learning agents **326** observations (e.g., the determined dose **320,** and the DVH curves **324,** the predicted three-dimensional dose distribution **306** and its associated DVH curves **310**)). The reinforcement learning agents **326** can decide which action to take (e.g., which objective to modify and by how much) based on their internal policies and rules. The reinforcement learning agents **326** can do so based on the differences, such as proportional to the differences. The reinforcement learning agents **326** can determine a second cost value based on the adjusted weights and/or objectives.

The reinforcement learning agents **326** can iteratively repeat **316-328** until determining a set of weighted objectives that converges. The reinforcement learning agents **326** can do so, for example, by comparing the first cost value and the second cost value to determine a difference in cost values. The reinforcement learning agents **326** can compare the difference to a threshold. Responsive to determining that the difference exceeds or otherwise does not satisfy the threshold, the reinforcement learning agents **326** can determine another three-dimensional dose distribution and DVH curves, including based on contours **330** of any newly added structures (e.g., around hot and/or cold regions in the three-dimensional space representing the patient). The reinforcement learning agents **326** can determine a difference between the newly determined three-dimensional dose distribution or DVH curves and the predicted three-dimensional dose distribution **306** or DVH curves **310**. The reinforcement learning agents **326** can adjust the weighted objectives of the cost function again and determine a third cost value for the twice-adjusted weighted objectives. The reinforcement learning agents **326** can repeat this process until determining a difference between two sequentially determined cost values is less than the threshold or at least a defined number of sequentially determined differences are less than the threshold.

The analytics server **110a** can use the final set of objectives to generate a radiotherapy treatment plan for the patient. In some cases, the final set of objectives may make up the radiotherapy treatment plan for the patient. The analytics server can store the radiotherapy treatment plan in memory with an identifier of the patient and/or transmit the radiotherapy treatment plan to a computing device or radiotherapy treatment machine to use for treatment.

**FIG. 4** illustrates a process flow diagram for training a reinforcement learning agent, according to an embodiment. The method **400** can be performed to train a reinforcement learning agent, such as the reinforcement learning agent **111.** The method **400** may include steps **402-412.** However, other embodiments may include additional or alternative steps or may omit one or more steps altogether. The method **400** is described as being executed by a server, such as the analytics server described in **FIG. 1.** However, one or more steps of the method **400** may be executed by any number of computing devices operating in the distributed computing system described in **FIG. 1.** For instance, one or more computing devices may locally perform part or all of the steps described in **FIG. 4.**

For example, at step **402,** the analytics server can execute a dose prediction machine learning model. The analytics server can execute the dose prediction machine learning model using treatment attributes for a patient (e.g., using treatment attributes for the patient as input). In one example, the analytics server can execute the dose prediction machine learning model using one or more treatment attributes (e.g., second treatment attributes) for a patient (e.g., a second patient) to generate a predicted three-dimensional dose distribution (e.g., a second predicted three-dimensional dose distribution) for the patient. The analytics server can execute the dose prediction machine learning model in the same or a similar manner to the manner of execution described with reference to step **202.**

At step **404,** the analytics server can generate a set of weighted dose-volume objectives (e.g., a second set of weighted dose-volume objectives) of a cost function (e.g., a second cost function). The analytics server can do so using the predicted three-dimensional dose distribution and structure contours that the analytics server receives or identifies for the patient. The analytics server can generate the set of weighted dose-volume objectives in the same or a similar manner to the manner of execution described with reference to step **204.**

At step **406,** the analytics server can determine a three-dimensional dose distribution (e.g., a third three-dimensional dose distribution) that reduces (e.g., minimizes) a cost value (e.g., a third cost value) of the cost function. The analytics server can do so using a plan optimization algorithm. For example, the analytics server can use an optimization algorithm on a cost function containing the objectives that the analytics server generated for the patient using the dose prediction machine learning model. The analytics server can use the optimization algorithm on the cost function containing the objectives to generate the three-dimensional dose distribution that reduces the cost value of the cost function. The analytics server can determine the three-dimensional dose distribution in the same or a similar manner to the manner of execution described with reference to step **206.**

At step **408,** the analytics server can determine a reward value. The analytics server can determine the reward value at least according to a difference between the three-dimensional dose distribution and the predicted three-dimensional dose distribution. For example, the analytics server can compare the individual dosage values of the three-dimensional dose distribution and the predicted three-dimensional dose distribution that correspond to the same structures or spaces of the patient. The analytics server can use a function, such as a distance function or a weighted distance function, to determine a difference or distance between the two three-dimensional dose distributions. The analytics server can determine the difference in the same or a similar manner to the manner as the reinforcement learning agent as described with reference to step **208.** The analytics server can determine a reward value based on and/or proportional to the difference. For example, the analytics server can determine the reward value to be higher the smaller the difference or higher the higher the difference.

In some cases, the analytics server can use other factors to determine reward values in addition to or instead of the difference between the predicted three-dimensional dose distribution and the three-dimensional dose distribution. For example, the analytics server can use a set of rules to adjust the reward value. For instance, the analytics server can determine the reward value based on whether individual structures receive at least a target dosage, are above a target dosage, or are within a range of a target dosage in the three-dimensional dose distribution. In another example, the analytics server can determine the reward value based on whether structures labeled as not to receive any dosage, such as organs at risk that are far away from a target structure of the patient, receive any dosage. The reinforcement learning agent can determine the reward value using any type of rule.

In some cases, the analytics server can use a step-based reward value determination. For example, the analytics server may first determine the difference between the predicted three-dimensional dose distribution and the three-dimensional dose distribution. The analytics server may compare the difference to a threshold (e.g., a distance threshold or a difference threshold). Responsive to determining that the difference exceeds or otherwise does not satisfy the threshold, the analytics server may only use the difference to determine the reward value. However, responsive to determining that the difference is less than or otherwise satisfies the threshold, the analytics server can use one or more rules of a set of criteria to adjust (e.g., increase or decrease) the reward value. Accordingly, the analytics server can initially generate reward values towards generating a three-dimensional dose distribution which resembles the predicted three-dimensional dose distribution. Subsequently, the analytics server can generate reward values that may be smaller, but still positive, if the analytics server can generate a more effective three-dimensional dose distribution, for example a more effective three-dimensional dose distribution than the predicted three-dimensional dose distribution (e.g., a three-dimensional dose distribution that has a lower total dosage or that has lower dosage in defined or defined areas or a structures of a patient).

At step **410,** the analytics server can adjust the set of weighted dose-volume objectives. The analytics server can use the reinforcement learning agent to do so. The reinforcement learning agent can adjust the set of weighted dose-values in a similar manner to the manner described with reference to step **210.** For example, the reinforcement learning agent can adjust the set of weighted dose-volume objectives by adjusting the objectives and/or the weights associated with the objectives. The reinforcement learning agent can adjust the set of weighted dose-volume objectives according to an internal policy of the reinforcement learning agent. In cases in which the weights were assigned according to a ranking system, the reinforcement learning agent may adjust the weights while preserving the relative order of the objective weights. The analytics server can use the reinforcement learning agent to adjust the set of weighted dose-volume objectives in any manner.

At step **412,** the analytics server can train the reinforcement learning agent. The analytics server can train the reinforcement learning agent based on the reward value determined at step **408.** In some cases, the analytics server can perform step **412** prior to step **410.** For example, the analytics server can train the reinforcement learning agent by updating the reinforcement learning agent's current policy and/or learning algorithm, such as using Q-learning, policy gradients, and/or actor-critic methods based on the reward value. The analytics server can update the policy of the reinforcement learning agent using any of such methods to reinforce the reinforcement learning agent making adjustments to weighted objectives (e.g., actions) that bring the weighted objectives of a radiotherapy treatment plan closer to objectives that result in personalized predicted three-dimensional dose distributions for patients and/or that improve upon such personalized predicted three-dimensional dose distributions. In cases in which the reward is negative or is a penalty, the analytics server can update the policy to avoid actions that would result in adjustments to weighted objectives that would result in worse three-dimensional dose distributions and/or weighted objectives that would result in three-dimensional dose distributions that are further from predicted dose distributions. Accordingly, the analytics server can train the reinforcement learning agent to make more accurate adjustments to weighted objectives of a radiotherapy treatment plan when iteratively adjusting the weighted objectives, thus reducing the number of adjustments and/or amount of iterative processing that may be required when generating an optimized radiotherapy treatment plan.

The analytics server can repeat steps **404-412** any number of times and/or until the analytics server generates a set of weighted objectives that converge the cost function, as described herein. With each iteration, the analytics server can further train the reinforcement learning agent based on a new reward value, further improving the policy of the reinforcement learning agent. The analytics server can perform the method **400** for any number of patients to train the reinforcement learning agent, in some cases training the reinforcement learning agent until the reinforcement learning agent is accurate to a threshold. The analytics server can implement the reinforcement learning agent (e.g., in the manner described with reference to the method **200),** in some cases responsive to determining the reinforcement learning agent is accurate to the threshold.

The analytics server may similarly use the reinforcement learning agent and dose prediction machine learning model to generate radiotherapy treatment plans for patients over time. In doing so, the analytics server may gradually train the reinforcement learning agent for each radiotherapy treatment plan based on the reward values generated during the iterative process. Accordingly, the analytics server may gradually train the reinforcement learning agent to improve the actions the reinforcement learning agent takes in adjusting sets of weighted objectives to reduce the number of iterations of adjustments the reinforcement learning agent takes when generating sets of weighted objectives before the sets of weighted objectives converge. Thus, the analytics server can gradually train the reinforcement learning agent to require fewer processing resources and operate with less latency when generating objectives for radiotherapy treatment plans.

Advantageously, by implementing the systems and methods described herein, a computer can train and use one or more reinforcement learning agents to generate radiotherapy treatment plan with less latency and using less processing resources. The computer can do so by using a dose prediction machine learning model that can generate a predicted personalized baseline three-dimensional dose distributions for patients. For example, during a training stage, the reinforcement learning agents can be trained based on differences between three-dimensional dose distributions generated based on weighted objectives that the reinforcement learning models generate and predicted personalized baseline three-dimensional dose distributions. The reinforcement learning agents can be rewarded based on the differences such that the reinforcement learning agents can identify three-dimensional dose distributions that follow any defined criteria while remaining close to the predicted three-dimensional dose distributions. In doing so, the reinforcement learning agents can be trained to use fewer and fewer iterations to determine objectives of radiotherapy treatment plans during an inference stage, which can facilitate the reinforcement learning agents generating the correct objectives faster and using fewer computing resources.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1.  A method comprising:

    executing, by a processor, a dose prediction machine learning model using one or more treatment attributes for a patient to generate a predicted three-dimensional dose distribution for the patient;
    generating, by the processor, a set of weighted dose-volume objectives of a cost function based on the predicted three-dimensional dose distribution for the patient;
    determining, by the processor, a first three-dimensional dose distribution that reduces a first cost value of the cost function;
    determining, by the processor, a difference between the first three-dimensional dose distribution and the predicted three-dimensional dose distribution;
    adjusting, by the processor using a reinforcement learning agent, the set of weighted dose-volume objectives of the cost function based on the difference between the first three-dimensional dose distribution and the predicted three-dimensional dose distribution;
    determining, by the processor, a second three-dimensional dose distribution that reduces a second cost value based on the adjusted set of weighted dose-volume objectives of the cost function; and
    responsive to determining a difference between the first cost value and the second cost values satisfies a threshold, generating, by the processor, a radiotherapy treatment plan for the patient based on the adjusted set of weighted dose-volume objectives.

2.  The method of claim 1, further comprising:

    executing, by the processor, the dose prediction machine learning model using one or more second treatment attributes for a second patient to generate a second predicted three-dimensional dose distribution for the second patient;
    generating, by the processor, a second set of weighted dose-volume objectives of a second cost function based on the second predicted three-dimensional dose distribution for the second patient;
    determining, by the processor, a third three-dimensional dose distribution that reduces a third cost value of the second cost function;
    determining, by the processor, a second difference between the third three-dimensional dose distribution and the second predicted three-dimensional dose distribution;
    determining, by the processor, a reward value at least according to the difference between the third three-dimensional dose distribution and the second predicted three-dimensional dose distribution; and

training, by the processor, the reinforcement learning agent based on the reward value.

3.  The method of claim 2, further comprising:

    receiving, by the processor, third one or more treatment attributes of a third radiotherapy treatment plan for a third patient;
    generating, by the processor, a third set of weighted dose-volume objectives based on the third one or more treatment attributes for the third patient;
    executing, by the processor, the trained reinforcement learning agent to adjust the third set of weighted dose-volume objectives; and
    generating, by the processor, a third radiotherapy treatment plan for the third patient based on the third adjusted set of weighted dose-volume objectives.

4.  The method of claim 2 or claim 3, wherein determining the reward value comprises determining, by the processor, the reward value based on a comparison of the difference between the third three-dimensional dose distribution and a second threshold.

5.  The method of any of claim 2 to claim 4, wherein determining the reward value comprises:

    applying, by the processor, a set of criteria to the third three-dimensional dose distribution; and
    determining, by the processor, the reward value based on the application of the set of criteria to the third three-dimensional dose distribution.

6.  The method of any of claim 2 to claim 5 wherein determining the reward value comprises:

    responsive to determining the third three-dimensional dose distribution is within the threshold of the second predicted three-dimensional dose distribution, applying, by the processor, a set of criteria to the third three-dimensional dose distribution; and
    determining, by the processor, the reward value based on the application of the set of criteria to the third three-dimensional dose distribution.

7.  The method of any preceding claim, wherein generating the set of weighted dose-volume objectives of the cost function comprises assigning, by the processor, one or more weights according to a stored template of weights that indicates weights to apply to different structures of the patient;
    and/or:
    wherein generating the set of weighted dose-volume objectives of the cost function comprises assigning, by the processor, one or more weights according to a stored a ranked list of targets for the radiotherapy treatment plan.

8.  The method of any preceding claim, wherein adjusting the set of weighted dose-volume objectives comprises inserting, by the processor using the reinforcement learning agent, one or more second objectives and corresponding weights into the set of weighted dose-volume objectives, the one or more second objectives corresponding to different structures of the patient;
    and/or:
    wherein each objective of the set of weighted dose-volume objectives corresponds to a different structure within the patient and a different reinforcement learning agent of a plurality of reinforcement learning agents, and
    wherein adjusting the set of weighted dose-volume objectives of the cost function comprises adjusting, by the processor, the set of weighted dose-volume objectives using the plurality of reinforcement learning agents.

9.  A system comprising:

    one or more processors coupled with memory, the memory comprising instructions that, when executed by the one or more processors, cause the one or more processors to:
    execute a dose prediction machine learning model using one or more treatment attributes for a patient to generate a predicted three-dimensional dose distribution for the patient;
    generate a set of weighted dose-volume objectives of a cost function based on the predicted three-dimensional dose distribution for the patient;
    determine a first three-dimensional dose distribution that reduces a first cost value of the cost function;
    determine a difference between the first three-dimensional dose distribution and the predicted three-dimensional dose distribution;

adjust, using a reinforcement learning agent, the set of weighted dose-volume objectives of the cost function based on the difference between the first three-dimensional dose distribution and the predicted three-dimensional dose distribution;

determine a second three-dimensional dose distribution that reduces a second cost value based on the adjusted set of weighted dose-volume objectives of the cost function; and

responsive to determining a difference between the first cost value and the second cost values satisfies a threshold, generate a radiotherapy treatment plan for the patient based on the adjusted set of weighted dose-volume objectives.

10. The system of claim 9, wherein the instructions further cause the one or more processors to:

execute the dose prediction machine learning model using one or more second treatment attributes for a second patient to generate a second predicted three-dimensional dose distribution for the second patient;

generate a second set of weighted dose-volume objectives of a second cost function based on the second predicted three-dimensional dose distribution for the second patient;

determine a third three-dimensional dose distribution that reduces a third cost value of the second cost function;

determine a second difference between the third three-dimensional dose distribution and the second predicted three-dimensional dose distribution;

determine a reward value at least according to the difference between the third three-dimensional dose distribution and the second predicted three-dimensional dose distribution; and

train the reinforcement learning agent based on the reward value.

11. The system of claim 10, wherein the instructions further cause the one or more processors to:

receive third one or more treatment attributes of a third radiotherapy treatment plan for a third patient;

generate a third set of weighted dose-volume objectives based on the third one or more treatment attributes for the third patient;

execute the trained reinforcement learning agent to adjust the third set of weighted dose-volume objectives; and

generate a third radiotherapy treatment plan for the third patient based on the third adjusted set of weighted dose-volume objectives.

12. The system of claim 10 or claim 11, wherein the instructions cause the one or more processors to determine the reward value by determining the reward value based on a comparison of the difference between the third three-dimensional dose distribution and a second threshold.

13. The system of any of claim 10 to claim 12, wherein the instructions cause the one or more processors to determine the reward value by:

applying a set of criteria to the third three-dimensional dose distribution; and

determining the reward value based on the application of the set of criteria to the third three-dimensional dose distribution;

and/or:

wherein the instructions cause the one or more processors to determine the reward value by

responsive to determining the third three-dimensional dose distribution is within the threshold of the second predicted three-dimensional dose distribution, applying a set of criteria to the third three-dimensional dose distribution; and

determining the reward value based on the application of the set of criteria to the third three-dimensional dose distribution.

14. The system of any of claim 9 to claim 13, wherein the instructions cause the one or more processors to generate the set of weighted dose-volume objectives of the cost function by assigning one or more weights according to a stored template of weights that indicates weights to apply to different structures of the patient;

and/or:

wherein the instructions cause the one or more processors to generate the set of weighted dose-volume objectives of the cost function by assigning one or more weights according to a stored a ranked list of targets for the radiotherapy treatment plan.

15. The system of any of claim 9 to claim 14, wherein the instructions cause the one or more processors to adjust the set of weighted dose-volume objectives by inserting, using the reinforcement learning agent, one or more second objectives and corresponding weights into the set of weighted dose-volume objectives, the one or more second objectives corresponding to different structures of the patient;
and/or:
wherein each objective of the set of weighted dose-volume objectives corresponds to a different structure within the patient and a different reinforcement learning agent of a plurality of reinforcement learning agents, and
wherein the instructions cause the one or more processors to adjust the set of weighted dose-volume objectives of the cost function by adjusting the set of weighted dose-volume objectives using the plurality of reinforcement learning agents.

**FIG. 1**

Execute a dose prediction machine learning model using one or more treatment attributes of a radiotherapy treatment plan for a patient to generate a predicted three-dimensional dose distribution for the patient. **202**

$\downarrow$

Generate a set of weighted dose-volume objectives of a cost function based on the predicted three-dimensional dose distribution for the patient. **204**

$\downarrow$

Determine a first three-dimensional dose distribution that reduces a first cost value of the cost function. **206**

$\downarrow$

Determine a difference between the first three-dimensional dose distribution and the predicted three-dimensional dose distribution. **208**

$\downarrow$

Adjust the set of weighted dose-volume objectives of the cost. **210**

$\downarrow$

Determine a second three-dimensional dose distribution that reduces a second cost value based on the adjusted set of weighted dose-volume objectives of the cost function. **212**

$\downarrow$

Difference satisfies threshold? **214**

N

Y $\downarrow$

Generate a radiotherapy treatment plan for the patient based on the adjusted set of weighted dose-volume objectives. **216**

# FIG. 2

EP 4 741 006 A1

**FIG. 3**

EP 4 741 006 A1

400

Execute a dose prediction machine learning model using one or more treatment attributes for a patient to generate a predicted three-dimensional dose distribution for the patient. **402**

Generate a set of weighted dose-volume objectives of a cost function based on the predicted three-dimensional dose distribution for the patient. **404**

Determine a three-dimensional dose distribution that reduces a cost value of the cost function. **406**

Determine a reward value at least according to the difference between the three-dimensional dose distribution and the predicted three-dimensional dose distribution. **408**

Adjust the weighted dose-volume objectives. **410**

Train the reinforcement learning agent based on the reward value. **412**

# FIG. 4

EP 4 741 006 A1

Europäisches
Patentamt

European
Patent Office

Office européen
des brevets

## EUROPEAN SEARCH REPORT

**Application Number**

EP 25 21 3874

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2024/001149 A1 (ZHAO KEJUN [CN] ET AL) 4 January 2024 (2024-01-04) * paragraphs [0005], [0016], [0077] - [0079], [0108] - [0115], [0137] - [0141] * | 1-15 | INV. A61N5/10 ADD. G06N20/00 G16H20/40 |
| A | US 2023/087944 A1 (HAKALA MIKKO [FI] ET AL) 23 March 2023 (2023-03-23) * paragraphs [0005] - [0010], [0046] - [0048], [0068] - [0079] * | 1-15 | |
| A | DURSUN PINAR ET AL: "Automated VMAT treatment planning using sequential convex programming: algorithm development and clinical implementation", 20230719, vol. 68, no. 15, 19 July 2023 (2023-07-19) , XP020555902, DOI: 10.1088/1361-6560/ACE09E [retrieved on 2023-07-19] * par. 2.1, 2.4 * | 1-15 | |

**TECHNICAL FIELDS SEARCHED (IPC)**

A61N
G06E
G16H
G06N

The present search report has been drawn up for all claims

| Place of search | Date of completion of the search | Examiner |
|---|---|---|
| Munich | 25 March 2026 | Lohmann, Stefan |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
..........................................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 25 21 3874

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2026

| Patent document cited in search report | Publication date | Patent family member(s) | Publication date |
|---|---|---|---|
| US 2024001149 A1 | 04-01-2024 | NONE | |
| US 2023087944 A1 | 23-03-2023 | CN 117999612 A | 07-05-2024 |
| | | EP 4405976 A1 | 31-07-2024 |
| | | US 2023087944 A1 | 23-03-2023 |
| | | WO 2023043663 A1 | 23-03-2023 |

EPO FORM P0459

For more details about this annex : see Official Journal of the European Patent Office, No. 12/82